# EUROPÄISCHE PATENTSCHRIFT

(11) **EP 3 734 270 B1**
(45) Veröffentlichungstag und Bekanntmachung des Hinweises auf die Patenterteilung: **14.01.2026**
(21) Anmeldenummer: 20172279.0
(22) Anmeldetag: 30.04.2020
(51) Int. Cl.: G01N 33/12, G01N 27/02, F24C 7/08

(54) **SYSTEM UND VERFAHREN ZUR ERMITTLUNG VON INFORMATION IN BEZUG AUF EIN NAHRUNGSMITTEL**
SYSTEM AND METHOD FOR DETERMINING INFORMATION RELATED TO A FOOD PRODUCT
SYSTÈME ET PROCÉDÉ DE DÉTERMINATION DES INFORMATIONS CONCERNANT UNE DENRÉE ALIMENTAIRE

(30) Priorität: 03.05.2019 DE 102019206394
(43) Veröffentlichungstag der Anmeldung: 04.11.2020
(73) Patentinhaber: BSH Hausgeräte GmbH, 81739 München (DE)
(72) Erfinder: Söllner, Christoph, 81475 München (DE); Richter, Hoang, 83339 Chieming (DE)

(56) Entgegenhaltungen:
- EP-A1- 2 890 218
- EP-A1- 2 907 372
- EP-A2- 1 253 423
- WO-A1-2018/165671
- CN-Y- 200 989 899
- DE-A1- 102009 026 957

## Beschreibung

Die Erfindung betrifft ein System und ein entsprechendes Verfahren, mit denen in effizienter Weise Information in Bezug auf die Qualität und/oder in Bezug auf die empfohlene Verarbeitung eines Nahrungsmittels ermittelt werden kann.

In einem Supermarkt können Nahrungsmittel mit unterschiedlicher Qualität gekauft werden. Beispielsweise kann die Qualität von Fleisch, die in einem Supermarkt angeboten wird, variieren. Für einen Laien sind die Bestandteile eines Stücks Fleisch, wie Wassergehalt, Fettgehalt oder Proteinanteil, optisch meist nicht bewertbar. Die Zusammensetzung eines Nahrungsmittels hat typischerweise Auswirkungen darauf, wie das Nahrungsmittel am besten zubereitet, insbesondere gegart, wird.

Aus der EP 2 890 218 A1 ist ein Kochgerät mit zwei Elektroden zur Ermittlung einer Impedanz eines Garguts vorbekannt. Die EP 1 253 423 A2 beschreibt ein Messverfahren zur Untersuchung eines biologischen Mediums. EP 2 907 372 A1 beschäftigt sich mit einem Feuchtigkeitssensor für geerntetes Getreide. Die CN 200 989 899 Y offenbart ein Gerät zur Einschätzung einer Qualität von Getreidekörnern. WO 2018/165671 A1 beschreibt ein System zum Bestimmen einer internen Eigenschaft eines Nahrungsmittels.

Das vorliegende Dokument befasst sich mit der technischen Aufgabe, es einem Nutzer zu ermöglichen, in komfortabler und präziser Weise Information in Bezug auf ein Nahrungsmittel zu ermitteln und ggf. bei der Verarbeitung des Nahrungsmittels zu verwenden.

Die Aufgabe wird jeweils durch jeden einzelnen der Gegenstände der unabhängigen Patentansprüche gelöst. Vorteilhafte Ausführungsformen sind insbesondere in den abhängigen Patentansprüchen definiert, in nachfolgender Beschreibung beschrieben oder in der beigefügten Zeichnung dargestellt.

Das erfindungsgemäße System zur Ermittlung von Information in Bezug auf ein Nahrungsmittel umfasst eine Vorrichtung. Die Vorrichtung kann ausgebildet sein, um von einem Nutzer bei Bedarf auf einer Arbeitsplatte einer Küche abgestellt zu werden. Die Vorrichtung umfasst eine Messfläche zur Ablage eines Nahrungsmittels. Die Messfläche kann durch die Oberseite eines Gehäuses der Vorrichtung gebildet werden. Die Messfläche kann z.B. eine Größe von 30cm x 30cm (oder weniger) aufweisen.

Des Weiteren umfasst die Vorrichtung zumindest zwei an der Messfläche angeordnete Messelektroden, zwischen denen ein elektrisch isolierender Isoliersteg verläuft. Die einzelnen (flächigen) Messelektroden können dabei jeweils einen Teil der Messfläche bedecken. Die einzelnen Messelektroden können parallel zu der Messfläche verlaufen. Die Position der Messelektroden kann an der Messfläche optisch angezeigt sein (z.B. durch Markierungen), um es einem Nutzer zu ermöglichen, ein Nahrungsmittel (z.B. ein Stück Fleisch) in präziser Weise derart auf der Messfläche abzulegen, dass das Nahrungsmittel zumindest zwei Messelektroden (möglichst gleichmäßig bzw. zu gleichen Anteilen) bedeckt.

Die Messfläche kann ausgestaltet sein, um eine gleichmäßige Platzierung eines Nahrungsmittels auf der Messfläche zu begünstigen, beispielsweise durch eine oder mehrere Vertiefungen in der Mitte, in welche die Messelektroden formfolgend und/oder formschlüssig angebracht sind. Mit anderen Worten, die Messfläche kann nicht-eben und/oder gekrümmt sein. Die Messfläche kann derart ausgebildet sein, dass ein möglichst großer Teil der Oberfläche eines Nahrungsmittels die Messfläche berührt. Die Messelektroden können an die Form der Messfläche angepasst sein.

Die Vorrichtung umfasst eine Steuereinheit, die eingerichtet ist, anhand der Messelektroden Sensordaten in Bezug auf das auf der Messfläche abgelegte Nahrungsmittel zu erfassen. Die Sensordaten können z.B. ein oder mehrere Messwerte in Bezug auf die konduktive und/oder die dielektrische Leitfähigkeit des Nahrungsmittels umfassen. Alternativ oder ergänzend können die Sensordaten Messwerte für eine Vielzahl von unterschiedlichen Frequenzen einer an den Messelektroden angelegten Wechselspannung umfassen. Insbesondere kann durch die Messwerte die konduktive und/oder dielektrische Leitfähigkeit des Nahrungsmittels für eine Vielzahl von Frequenzen angezeigt werden.

Die Steuereinheit kann z.B. eingerichtet sein, für eine Vielzahl von unterschiedlichen Frequenzen (z.B. für unterschiedliche Frequenzen aus einem bestimmten Frequenzbereich zwischen 50Hz und 20kHz), eine Wechselspannung an zumindest einem Teil der Messelektroden der Vorrichtung zu bewirken (z.B. jeweils paarweise an zwei Messelektroden). Es kann dann jeweils ein Messwert in Bezug auf die Amplitude der Wechselspannung als Teil der Sensordaten erfasst werden. So kann z.B. ein Frequenzverlauf von Messwerten für unterschiedliche Frequenzen erfasst werden.

Außerdem ist die Steuereinheit der Vorrichtung eingerichtet zu veranlassen, dass auf Basis der Sensordaten Nahrungsmittel-Information in Bezug auf die Verarbeitung des Nahrungsmittels ermittelt wird. Die Nahrungsmittel-Information wird von einer externen Zentraleinheit (z.B. von einem Backend-Server) ermittelt. Die Nahrungsmittel-Information zeigt an, wie ein Hausgerät bei der Verarbeitung (z.B. beim Garen) des Nahrungsmittels einzustellen ist. Zur Ermittlung der Nahrungsmittel-Information kann z.B. eine Frequenzanalyse des Frequenzverlaufs der Messwerte durchgeführt werden. Alternativ oder ergänzend kann der Frequenzverlauf der Messwerte mit Referenzdaten verglichen werden, um die Nahrungsmittel-Information zu ermitteln. Alternativ oder ergänzend kann ein maschinen-erlernter Klassifikator (z.B. mit einem neuronalen Netzwerk) verwendet werden, um auf Basis der Sensordaten die Nahrungsmittel-Information zu ermitteln.

Die einzelnen Messelektroden weisen Kanten auf, wobei die Kanten einer Messelektrode in Summe eine Gesamt-Kantenlänge aufweisen. Die Kanten zumindest eines Teils der Messelektroden können an den Isoliersteg angrenzen. Die an den Isoliersteg angrenzenden Messelektroden können derart geformt sein, dass der an den Isoliersteg angrenzende Anteil der Gesamt-Kantenlänge zumindest einer Messelektrode größer als 25% ist. Mit anderen Worten, die Messelektroden können derart geformt sein, dass ein möglichst hoher Anteil der Kanten der Messelektroden an dem Isoliersteg angeordnet ist. Zu diesem Zweck können die Messelektroden z.B. dreiecksförmig sein. So kann die Güte der erfassten Sensordaten erhöht werden.

Die Vorrichtung umfasst eine Kommunikationseinheit die eingerichtet ist, über eine Kommunikationsverbindung mit der Zentraleinheit zu kommunizieren. Dabei kann eine drahtlose und/oder eine drahtgebundene Kommunikation ermöglicht werden. Die Steuereinheit ist eingerichtet die Sensordaten zur Ermittlung der Nahrungsmittel-Information über die Kommunikationseinheit an die externe Zentraleinheit zu senden. So kann in präziser Weise (z.B. unter Verwendung von relativ hohen Computer-Ressourcen) die Nahrungsmittel-Information durch die Zentraleinheit ermittelt werden. Die Nahrungsmittel-Information kann dann ggf. zurück zu der Vorrichtung gesendet werden.

Die Vorrichtung kann eine Benutzerschnittstelle (z.B. mit einem Bildschirm und/oder mit ein oder mehreren Bedienelementen) umfassen. Die Steuereinheit kann eingerichtet sein, die Nahrungsmittel-Information über die Kommunikationseinheit von der externen Einheit zu empfangen. Die Benutzerschnittstelle der Vorrichtung kann dann veranlasst werden, die Nahrungsmittel-Information an einen Nutzer der Vorrichtung auszugeben (z.B. auf dem Bildschirm anzuzeigen). So kann die Nahrungsmittel-Information in komfortabler Weise bereitgestellt werden.

Die Vorrichtung kann einen Gewichtssensor umfassen, der eingerichtet ist, Gewichtsdaten in Bezug auf das auf der Messfläche abgelegte Nahrungsmittel zu erfassen. Auf Basis der Gewichtsdaten kann z.B. erkannt werden, ob ein Nahrungsmittel auf der Messfläche abgelegt wurde oder nicht. Die Steuereinheit kann eingerichtet sein, zu veranlassen, dass die Nahrungsmittel-Information auf Basis bzw. unter Berücksichtigung der Gewichtsdaten ermittelt wird. So kann die Güte der ermittelten Nahrungsmittel-Information erhöht werden. Alternativ oder ergänzend kann die Steuereinheit eingerichtet sein, das Erfassen der Sensordaten in Abhängigkeit von den Gewichtsdaten automatisch zu starten. So kann der Komfort der Vorrichtung für einen Nutzer erhöht werden.

Die Steuereinheit kann eingerichtet sein, anhand einer Nutzereingabe an der Benutzerschnittstelle einen Nahrungsmitteltyp des abgelegten Nahrungsmittels aus einer Mehrzahl von unterschiedlichen Nahrungsmitteltypen zu ermitteln. Insbesondere kann es einem Nutzer ermöglicht werden, den Typ des Nahrungsmittels, das auf die Messfläche gelegt wurde oder wird, aus einer List von unterschiedlichen Nahrungsmitteltypen auszuwählen. Die Nahrungsmittel-Information kann dann in besonders präziser Weise auf Basis bzw. unter Berücksichtigung des ermittelten Nahrungsmitteltyps ermittelt werden. Die Vorrichtung kann einen (ggf. wiederaufladbaren) elektrischen Energiespeicher umfassen, der eingerichtet ist, elektrische Energie für einen autarken Betrieb der Vorrichtung zu speichern. So kann der Komfort der Vorrichtung weiter erhöht werden.

Die Vorrichtung kann eine Vielzahl von Messelektroden (in diesem Dokument auch als Elektrodensegmente bezeichnet) umfassen. Die einzelnen Messelektroden können rasterförmig und/oder matrixförmig an der Messfläche angeordnet sein. Beispielsweise kann die Vorrichtung 20 oder mehr, 30 oder mehr, oder 40 oder mehr Messelektroden aufweisen, die (jeweils durch einen Isoliersteg voneinander isoliert) an der Messfläche angeordnet sind.

Die Steuereinheit kann eingerichtet sein, einen Teilbereich der Messfläche zu ermitteln, der von dem Nahrungsmittel bedeckt ist. Beispielsweise kann mittels der Bilddaten einer Kamera der Vorrichtung ermittelt werden, welcher Teil der Messfläche durch das abgelegte Nahrungsmittel bedeckt wird, und welcher Teil der Messfläche unbedeckt bleibt. Es kann dann in Abhängigkeit von dem ermittelten Teilbereich der Messfläche eine Teilmenge der Vielzahl von Messelektroden ausgewählt werden. Die Teilmenge kann alle und/oder nur die Messelektroden umfassen, die zumindest teilweise (oder die vollständig) von dem Nahrungsmittel bedecket werden.

Die Steuereinheit kann eingerichtet sein, die Sensordaten (ggf. ausschließlich) anhand der ermittelten Teilmenge von Messelektroden zu ermitteln. Durch die Auswahl einer Teilmenge von Messelektroden kann der Abdeckungsgrad der zur Messung verwendeten Messelektroden erhöht werden, wodurch wiederum die Güte der erfassten Sensordaten und somit die Güte der ermittelten Nahrungsmittel-Information erhöht wird.

Das erfindungsgemäße System zur Ermittlung von Information in Bezug auf ein Nahrungsmittel umfasst:
- die Zentraleinheit, insbesondere einen Server;
- ein elektronisches Anwendergerät, insbesondere ein Smartphone; und
- die oben beschriebene Vorrichtung.

Die Steuereinheit ist dazu eingerichtet, anhand der Messelektroden Sensordaten in Bezug auf das auf der Messfläche abgelegte Nahrungsmittel zu erfassen; und zu veranlassen, dass auf Basis der Sensordaten Nahrungsmittel-Information in Bezug auf eine Verarbeitung des Nahrungsmittels ermittelt wird, wobei die Nahrungsmittel-Information anzeigt, wie ein Hausgerät bei der Verarbeitung des Nahrungsmittels einzustellen ist, und die Sensordaten zur Ermittlung der Nahrungsmittel-Information über die Kommunikationseinheit an die Zentraleinheit gesendet werden.

Darüber hinaus ist die Zentraleinheit dafür eingerichtet ist, auf Basis der Sensordaten die Nahrungsmittel-Information in Bezug auf eine Verarbeitung des Nahrungsmittels zu ermitteln und die Nahrungsmittel-Information an das elektronische Anwendergerät zu senden.

Die Zentraleinheit kann eingerichtet sein, ein Hausgerät (z.B. einen Ofen oder einen Herd oder ein Kochfeld) zur Verarbeitung des Nahrungsmittels automatisch in Abhängigkeit von der Nahrungsmittel-Information zu betreiben. So kann die Güte der Verarbeitung eines Nahrungsmittels erhöht werden.

Wie bereits oben dargelegt, können die Sensordaten Messwerte für eine Vielzahl von unterschiedlichen Frequenzen einer an Messelektroden der Vorrichtung angelegten Wechselspannung umfassen. Die Zentraleinheit kann eingerichtet sein, anhand der Messwerte für unterschiedliche Frequenzen Mengeninformation für unterschiedliche Bestandteile des Nahrungsmittels zu ermitteln. Dabei können bestimmte unterschiedliche Frequenzen für jeweils unterschiedliche bestimmte Bestandteile des Nahrungsmittels relevant sein (z.B. Wasser, Fett, Proteine, etc.). Durch die Analyse der Messwerte für bestimmte Frequenzen, die für jeweils unterschiedliche Bestandteile relevant sind, kann die Nahrungsmittel-Information in besonders präziser Weise ermittelt werden.

Gemäß einem weiteren Aspekt umfasst die Erfindung ein Verfahren zur Ermittlung von Information in Bezug auf ein Nahrungsmittel mittels des oben beschriebenen Systems. Das Verfahren umfasst das Erfassen, anhand der Messelektroden, von Sensordaten in Bezug auf ein auf der Messfläche abgelegtes Nahrungsmittel; das Senden der Sensordaten zur Ermittlung der Nahrungsmittel-Information über die Kommunikationseinheit der Vorrichtung und das Internet an die Zentraleinheit; das Ermitteln auf Basis der Sensordaten von Nahrungsmittel-Information in Bezug auf eine Verarbeitung des Nahrungsmittels mittels der Zentraleinheit, wobei die Nahrungsmittel-Information anzeigt, wie ein Hausgerät bei der Verarbeitung des Nahrungsmittels einzustellen ist; und das Senden der Nahrungsmittel-Information von der Zentraleinheit an das elektronische Anwendergerät.

Im Weiteren wird die Erfindung anhand von in der beigefügten Zeichnung dargestellten Ausführungsbeispielen näher beschrieben. Dabei zeigen
Figur 1a ein Blockdiagramm eines Systems zur Ermittlung von Nahrungsmittel-Information in Bezug auf ein Nahrungsmittel;
Figur 1b eine beispielhafte Sensoreinheit in einer Ansicht von Oben;
Figur 1c eine weitere beispielhafte Sensoreinheit;
Figur 1d eine beispielhafte Sensoreinheit in einer Seitenansicht;
Figuren 2a und 2b beispielhafte Sensordaten einer Sensoreinheit;
Figur 3a eine beispielhafte Elektrodenmatrix für eine Sensoreinheit;
Figur 3b eine beispielhafte gekrümmte Messfläche; und
Figur 4 ein Ablaufdiagramm eines beispielhaften Verfahrens zur Ermittlung von Nahrungsmittel-Information in Bezug auf die Güte und/oder die weitere Verarbeitung eines Nahrungsmittels.

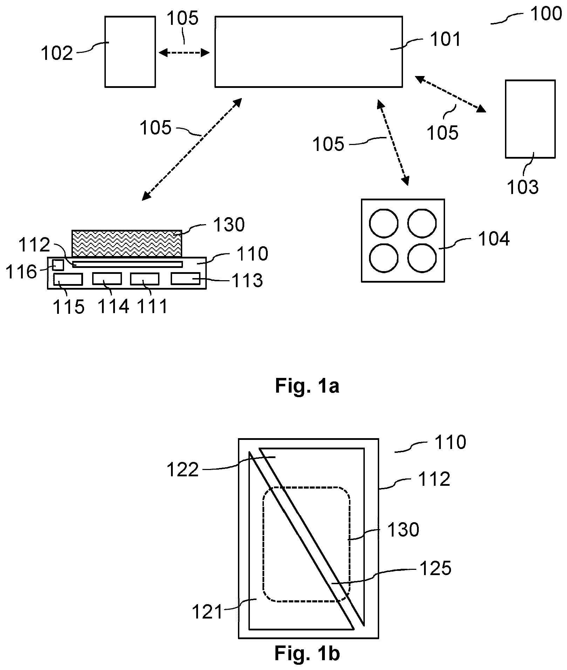

Wie eingangs dargelegt, befasst sich das vorliegende Dokument mit der komfortablen und zuverlässigen Ermittlung von Information in Bezug auf die Verarbeitung eines Nahrungsmittels, insbesondere von Fleisch. In diesem Zusammenhang zeigt Fig. 1a ein Blockdiagramm eines beispielhaften Systems 100, das es einem Nutzer ermöglicht, Information in Bezug auf ein Nahrungsmittel 130 zu ermitteln.

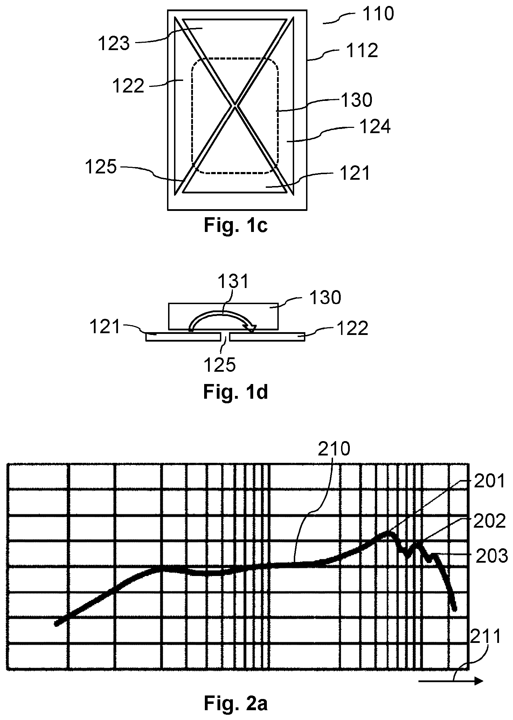

Das System 100 umfasst eine Sensoreinheit 110, die z.B. ausgebildet ist, auf eine Arbeitsplatte einer Küche gestellt zu werden. Die Sensoreinheit 110 wird in diesem Dokument auch allgemein als "Vorrichtung" bezeichnet. Die Sensoreinheit 110 umfasst eine Messfläche 112, auf die ein Nahrungsmittel 130 gelegt werden kann. Die Messfläche 112 umfasst, wie in den Figuren 1b und 1c dargestellt, zwei oder mehr flächige und/oder plattenförmige, elektrisch leitende, Messelektroden 121, 122, 123, 124, die jeweils über einen Spalt bzw. Steg 125 voneinander elektrisch isolierst sind. Ein Nahrungsmittel 130 (z.B. ein Stück Fleisch) kann derart auf die Messfläche 112 gelegt werden, dass das Nahrungsmittel 130 über mehreren Messelektroden 121, 122, 123, 124 angeordnet ist.

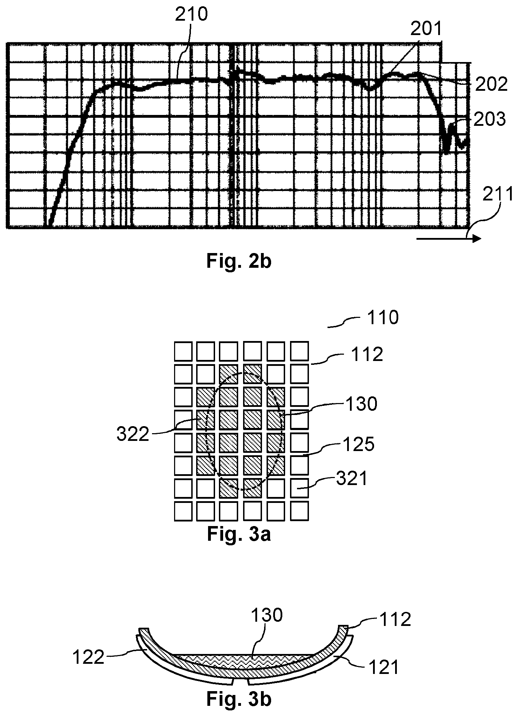

Zur Ermittlung von Sensordaten kann an die Messelektroden 121, 122, 123, 124 eine elektrische Wechselspannung mit einer bestimmten Frequenz angelegt werden. Beispielsweise können die Messelektroden 121, 122, 123, 124 einen Kondensator bilden, der Teil eines elektrischen Schwingkreises ist (z.B. eines LC-Schwingkreises). Es kann dann eine elektrische Wechselspannung mit einer bestimmten Frequenz an den elektrischen Schwingkreis angelegt werden, und es kann ermittelt werden, welche Spannungs- und/oder Stromamplitude sich an dem durch die Messelektroden 121, 122, 123, 124 gebildeten Kondensator einstellt. Eine derartige Messung kann für eine Vielzahl von unterschiedlichen Frequenzen durchgeführt werden. Die Figuren 2a und 2b zeigen unterschiedliche Frequenzgänge 210 (d.h. Sensordaten der Sensoreinheit 110 für eine Vielzahl von Frequenzen 211) für jeweils unterschiedliche Nahrungsmittel 130 (insbesondere für unterschiedliche Fleischsorten).

Die Sensoreinheit 110 umfasst ferner eine Steuereinheit 111 die eingerichtet ist, Funktionen der Sensoreinheit 110 zu steuern. Insbesondere ist die Steuereinheit 111 eingerichtet eine Kommunikationseinheit 113 der Sensoreinheit 110 zu veranlassen. die von der Sensoreinheit 110 erfassten Sensordaten in Bezug auf ein Nahrungsmittel 130 über eine (ggf. drahtlose) Kommunikationsverbindung 105 (z.B. über WLAN, 3G, 4G oder 5G) an eine Zentraleinheit 101 (z.B. an einen Backend-Server) zu senden.

Die Zentraleinheit 101 ist eingerichtet die Sensordaten (ggf. unter Verwendung von Daten aus einer Datenbank 102) auszuwerten, um Nahrungsmittel-Information in Bezug auf die (empfohlene) Verarbeitung des Nahrungsmittels 130 zu ermitteln. Ferner ist die Zentraleinheit 101 eingerichtet die ermittelte Nahrungsmittel-Information an ein elektronisches Anwendergerät 103 (z.B. an ein Smartphone) eines Nutzers des Systems 100 zu senden. Ergänzend kann die Information an ein Hausgerät 104 (z.B. an einen Ofen, an einen Herd und/oder an ein Kochfeld) gesendet werden, und/oder die Information kann dazu genutzt werden, automatisch ein Hausgerät 104 für die Verarbeitung des Nahrungsmittels 130 zu steuern (z.B. einzustellen).

Die Sensoreinheit 110 kann eine Benutzerschnittstelle 114 umfassen, die es einem Nutzer ermöglicht, eine Messung zu starten, Eingaben in Bezug auf eine Messung zu tätigen und/oder Ergebnisse einer Messung einzusehen. Beispielsweise kann es einem Nutzer ermöglicht werden, den Typ des Nahrungsmittels 130 auszuwählen und/oder einzugeben, für das eine Messung durchgeführt werden soll. Alternativ oder ergänzend kann die Benutzerschnittstelle 114 dazu genutzt werden, auf Basis der Sensordaten ermittelte Nahrungsmittel-Information in Bezug auf das Nahrungsmittel 130 auszugeben.

Des Weiteren kann die Sensoreinheit 110 eine elektrische Energiequelle 115 (z.B. eine Batterie und/oder einen Anschluss zu einer Stromversorgung) umfassen, über die die Sensoreinheit 110 mit elektrischer Energie versorgt wird. Ferner kann die Sensoreinheit 110 einen Gewichtssensor 116 umfassen, der eingerichtet ist, Gewichtsdaten in Bezug auf das Gewicht eines Nahrungsmittels 130 zu erfassen, das auf die Messfläche 112 gelegt wurde. Die Steuereinheit 111 kann eingerichtet sein, auf Basis der Gewichtsdaten automatisch eine Messung der Sensordaten zu starten.

Es werden somit ein System 100 sowie ein Verfahren beschrieben, mit denen die Qualität eines Nahrungsmittels 130 (insbesondere von Fleisch) zumindest qualitativ abgeschätzt werden kann, um einem Nutzer Information in Bezug auf ein oder mehrere Betriebsparameter (z.B. die Betriebstemperatur und/oder die Betriebsdauer) für den Betrieb eines Hausgeräts 104 und/oder ein oder mehrere Rezeptparameter (z.B. eine Mengeninformation) für ein Rezept zur Verarbeitung des Nahrungsmittels 130 zu präsentieren.

Das System 100 umfasst eine Sensoreinheit 110, die separat von einem Hausgerät 104 angeordnet ist, z.B. auf einem Küchentisch oder auf einer Arbeitsplatte. Die Sensoreinheit 110 weist ein Gehäuse aus einem elektrisch nicht leitfähigen Basismaterial (etwa Glas oder Kunststoff) auf. An einem planen Boden bzw. an einer planen Messfläche 112 sind von außen mindestens zwei flächige, elektrisch leitfähige, Elektroden 121, 122, 123, 124 angebracht. Die Elektroden 121, 122, 123, 124 sind gegeneinander elektrisch isoliert. Von jeder Elektrode 121, 122, 123, 124 führt eine einzelne isolierte Leitung (nicht dargestellt) zur einer Auswerteelektronik und/oder Steuereinheit 111.

In einer bevorzugten Ausgestaltung ist die Sensoreinheit 110 batteriebetrieben (z.B. mit einer (ggf. wiederaufladbaren) Batterie 115). Zur Anzeige und Signalisierung für einen Stand-Alone-Betrieb der Sensoreinheit 110 kann die Sensoreinheit 110 eine Benutzerschnittstelle 114 umfassen (z.B. mit einem Bildschirm und/oder einem Signalisierungsmodul, etwa einem Lautsprecher). Über die Benutzerschnittstelle 114 können ggf. Texte und/oder Grafiken (z.B. in unterschiedlichen Sprachen) darstellt werden. Des Weiteren kann die Benutzerschnittstelle 114 ein oder mehrere Bedienelemente (z.B. Eingabeknöpfe) umfassen, z.B. um den Typ eines Nahrungsmittels 130 einzugeben.

Ferner kann die Sensoreinheit einen Gewichtskraftsensor 116 (z.B. eine "Wägezelle") umfassen, um die Masse bzw. das Gewicht eines Nahrungsmittels 130 zu erfassen. Über eine Kommunikationseinheit 113 kann die Sensoreinheit 110 drahtlos und/oder kabelgebunden (ggf. über ein geeignetes Gateway) Daten mit einer Zentraleinheit 101 austauschen. Die Zentraleinheit 101 kann einen Algorithmus aufweisen, der mit Zusatzwissen aus einer Datenbank 102 ausgestattet ist und der eingerichtet ist, auf Basis der empfangenen Messwerte bzw. Sensordaten Information über ein vermessenes Nahrungsmittel 130 zu ermitteln (z.B. prozentuale Werte von Komponenten bzw. Bestandteilen des Nahrungsmittels 130 und/oder textuelle Empfehlungen in Bezug auf den Verzehr und/oder die Verarbeitung das Nahrungsmittel).

Die ermittelte Nahrungsmittel-Information wird auf dem elektronischen Anwendergerät 103 verfügbar gemacht. Ergänzend kann die Information über das Internet und/oder über verschiedene Schnittstellen und/oder einem Hausgerät 104 und/oder der Sensoreinheit 110 bereitgestellt werden. Beispielsweise kann die Information in einem Hausgerät 104 (z.B. einem Backofen oder Kochfeld) für einen Garprozess des Nahrungsmittels 130 verwendet werden.

Die Elektroden 121, 122, 123, 124 der Sensoreinheit 110 können auf der Oberfläche 112 (z.B. auf einer Glasoberfläche) in verschiedenen Formen und Anordnungen platziert werden. Dabei kann die Anzahl der Elektroden 121, 122, 123, 124 unterschiedlich sein. Die Anordnung und/oder die Anzahl von Elektroden 121, 122, 123, 124 kann derart gewählt werden, dass die kapazitive Kopplung (dargestellt durch das elektrische Feld 131 in Fig. 1d) zwischen mindestens zwei Elektroden 121, 122 durch eine möglichst große Abdeckung der Elektroden 121, 122 mit dem Nahrungsmittel 130 maximiert wird.

Unterschiedliche Nahrungsmittel 130 können jeweils unterschiedliche Größen aufweisen. Des Weiteren können die Nahrungsmittel 130 von einem Nutzer ggf. unterschiedlich auf der Messfläche 112 platziert werden. Eine relativ große Überdeckung kann z.B. erreicht werden, indem die Summe der Kantenlängen der Isolierstege 125 zwischen den Elektroden 121, 122, 123, 124 maximiert wird (wie in den Figuren 1b und 1c dargestellt). Durch einen schrägen Verlauf der ein oder mehreren Isolierstege 125 kann bei mittiger Anordnung des Nahrungsmittels 130 eine möglichst große Abdeckung jeder einzelnen Elektrode 121, 122, 123, 124 bewirkt werden. Die Auswerteelektronik 111 kann eingerichtet sein, Sensordaten an unterschiedlichen Elektroden 121, 122, 123, 124 und/oder an unterschiedlichen Paaren von Elektroden 121, 122, 123, 124 zu erfassen (z.B. an den zwei bzw. vier in den Figuren 1b und 1c dargestellten Elektroden 121, 122, 123, 124). So kann detaillierte Information über ein Nahrungsmittel 130 ermittelt werden. Die Elektroden 121, 122, 123, 124 können ggf. nur einen Teilbereich der Messfläche 112 bedecken.

Fig. 3a zeigt ein Beispiel für eine Messfläche 112, die eine Vielzahl (z.B. eine Matrix) von Elektrodensegmenten 321 aufweist. Die einzelnen Elektrodensegmente 321 können jeweils durch Isolierstege 125 elektrisch voneinander isoliert ein. Die Sensoreinheit 110 kann eingerichtet sein, zu ermitteln (z.B. anhand von Bilddaten einer Kamera, die auf die Messfläche 112 gerichtet ist (nicht dargestellt), wo ein Nahrungsmittel 130 auf der Messfläche 112 platziert ist. Es kann dann eine Teilmenge der Vielzahl von Elektrodensegmenten 321 aktiviert werden (dargestellt durch die schraffierten Elektrodensegmente 322), um zu bewirken, dass die für eine Messung verwendeten Elektrodensegmente 322 möglichst vollständig von dem Nahrungsmittel 130 bedeckt sind. So kann in präziser Weise ein möglichst großer Abdeckungsgrad der verwendeten (aktiveren) Elektrodensegmenten 322 durch das Nahrungsmittel 130 bewirkt werden (wodurch die Qualität der erfassten Sensordaten erhöht wird).

Beispielsweise umfasst die Teilmenge von aktivierten Elektrodensegmenten 322 N Elektrodensegmente 322 (z.B. N gleich 10 oder mehr). Aus den N Elektrodensegmenten 322 können dann jeweils unterschiedliche Paare von Elektrodensegmenten 322 gebildet werden (z.B. N*(N-1) Paare), um für das jeweilige Paar einen Frequenzverlauf 210 zu ermitteln. Alternativ oder ergänzend können die N Elektrodensegmente 322 zu zwei Teilgruppen von Elektrodensegmenten 322 (z.B. von jeweils N/2 Elektrodensegmenten 322) zusammengefasst werden, und die zwei Teilgruppen von Elektrodensegmenten 322 können als "Paar" von jeweils zusammengesetzten Messelektroden 121, 122 betrieben werden, um einen Frequenzverlauf 210 zu ermitteln. Die derart ermittelten Sensordaten (d.h. Frequenzverläufe 210) können ausgewertet werden, um in besonders präziser Weise Nahrungsmittel-Information zu ermitteln.

Die Messfläche 112 kann eine von einer Ebene abweichende Form aufweisen. Beispielsweise kann die Messfläche 112, wie beispielhaft in Fig. 3b dargestellt, gekrümmt sein. Fig. 3b zeigt einen beispielhaften Querschnitt durch eine gekrümmte Messfläche 112. Insbesondere kann die Messfläche 112 derart ausgebildet sein, dass durch die Messfläche 112 ein Behälter (z.B. eine Schale) zur Aufnahme eines Nahrungsmittels 130 gebildet wird. Die Messelektroden 121, 122 können an die Form der Messfläche 112 angepasst sein (und sich an die Messfläche 112 anschmiegen). Durch die Verwendung einer nicht-ebenen und/oder gekrümmten Messfläche 112 kann die Güte einer Messung weiter erhöht werden.

Zur Durchführung einer Messung kann der Nutzer über die Benutzerschnittstelle 114 angewiesen werden, die Nahrungsmittel-Probe 130 möglichst mittig mit der größten Fläche nach unten auf die Messfläche 112 zu legen. Des Weiteren kann optional der Nahrungsmitteltyp (z.B. "weißes Fleisch", "rotes Fleisch", etc.) über die Benutzerschnittstelle 114 eingegeben werden. Die Benutzerschnittstelle 114 kann ggf. über eine App auf dem Anwendergerät 103 des Nutzers bereitgestellt werden.

Auf Basis der Gewichtsdaten des optionalen Gewichtskraftsensors 116 und/oder in Reaktion auf einen anderen Trigger kann eine Messung gestartet werden. Dabei können Messungen für eine Vielzahl von unterschiedlichen Frequenzen 211 (z.B. für einen Frequenz-Sweep), ggf. in Abhängigkeit von dem Nahrungsmitteltyp, kreuz- und/oder paarweise für unterschiedliche Elektroden 121, 122, 123 124 bzw. Elektrodensegmente 322 durchgeführt werden.

Beispielsweise kann der durch ein Elektrodenpaar gebildete Kondensator aufgeladen werden, und es können Sensordaten in Bezug auf die Entladezeitdauer erfasst werden. Alternativ oder ergänzend kann ein Trickle-Charge Verfahren verwendet werden. Alternativ oder ergänzend können die Energie und/oder die Spannung bei einer bestimmten Frequenz 211 gemessen werden, um eine bestimmte Amplitude der Spannung und/oder des Stroms an dem durch ein Elektrodenpaar gebildeten Kondensator zu erreichen. Alternativ oder ergänzend kann die sich einstellende Spannungsamplitude bei einer bestimmten Frequenz 211 ermittelt werden. Die Figuren 2a und 2b zeigen beispielhafte Messwerte (d.h. Sensordaten) als Funktion der Frequenz 211. Die erfassten Sensordaten können an die Zentraleinheit 101 gesendet und von der Zentraleinheit 101 ausgewertet werden.

Charakteristisch für ein Nahrungsmittel 130 (z.B. "rotes Fleisch", "weißes Fleisch", etc.) ist, dass die unterschiedlichen Bestandteile des Nahrungsmittels 130 (z.B. Fett, Protein, Wasser, etc.) in jeweils einem unterschiedlichen bestimmten Frequenzbereich (ggf. bei einer bestimmten Elektrodenflächenanordnung und/oder Elektrodenfläche) aufgrund der kapazitiven Kopplung ein jeweiliges Maximum in dem Frequenzverlauf 210 aufweisen. In den Figuren 2a und 2b sind z.B. die Peaks 201, 202, 203 für den Wasseranteil, den Proteinanteil bzw. den Fettanteil dargestellt. Aus der Höhe der unterschiedlichen Peaks 201, 202, 203 kann auf die relative Menge der einzelnen, unterschiedlichen Bestandteile geschlossen werden. Das Fleisch aus Fig. 2a weist z.B. einen relativ hohen Fettanteil auf (und könnte daher als "durchwachsen" betrachtet werden), während das Fleisch aus Fig. 2b einen relativ niedrigen Fettanteil aufweist (und somit als "mager" betrachtet werden könnte).

Aus der Amplitude bzw. den Verhältnissen der Amplituden der Peaks 201, 202, 203 für die einzelnen Bestandteile kann der Algorithmus der Zentraleinheit 101 eine entsprechende numerische Abschätzung der Anteile der einzelnen Bestandteile, z.B. in Prozent, ermitteln. Die ermittelten Anteile der Bestandteile können dann an der Sensoreinheit 110, an einem elektronischen Anwendergerät 103 und/oder an einem Hausgerät 104 ausgegeben werden. Alternativ oder ergänzend können auf Basis der Anteile und auf Basis einer Lookup-Tabelle und/oder eines Expertensystems für einen Nutzer verständliche Aussagen in Bezug auf die Qualität eines Nahrungsmittels 130 ermittelt und ausgegeben werden.

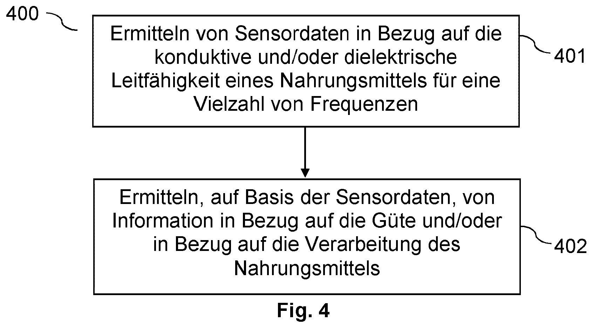

Fig. 4 zeigt ein Ablaufdiagramm eines beispielhaften Verfahrens 400 zur Ermittlung von Information in Bezug auf ein Nahrungsmittel 130 mittels einer Vorrichtung 110, die zumindest zwei an einer Messfläche 112 angeordnete (planare) Messelektroden 121, 122, 123, 124 umfasst, zwischen denen zumindest ein elektrisch isolierender Isoliersteg 125 verläuft.

Das Verfahren 400 umfasst das Erfassen 401, anhand der Messelektroden 121, 122, 123, 124, von Sensordaten in Bezug auf ein auf der Messfläche 112 abgelegtes Nahrungsmittel 130 (insbesondere in Bezug auf Fleisch, das auf der Messfläche 112 abgelegt wurde). Das Nahrungsmittel 130 bedeckt dabei zumindest teilweise den Isoliersteg 125 und zwei oder mehr der Messelektroden 121, 122, 123, 124. Zum Erfassen der Sensordaten kann eine Wechselspannung an zumindest einem Teil der Messelektroden 121, 122, 123, 124 angelegt werden. Ferner kann als Sensordaten eine Reaktion des Teils der Messelektroden 121, 122, 123, 124 (z.B. eine sich einstellende Spannungsamplitude) erfasst werden. Die Frequenz 211 der Wechselspannung kann in einem bestimmten Frequenzbereich (z.B. 50Hz bis 20kHz) variiert werden, um Messwerte für eine Vielzahl von unterschiedlichen Frequenzen 211 (insbesondere einen Frequenzverlauf 210 von Messwerten) zu erfassen.

Außerdem umfasst das Verfahren 400 das Veranlassen 402, dass auf Basis der Sensordaten Nahrungsmittel-Information in Bezug auf eine Verarbeitung des Nahrungsmittels 130 ermittelt wird. Die Nahrungsmittel-Information wird durch die externe Zentraleinheit 101 ermittelt. Zu diesem Zweck können die Messwerte für unterschiedliche Frequenzen 211 analysiert werden (insbesondere für typische Frequenzen 211 und/oder den sich einstellenden unterschiedlichen Impulsantworten für unterschiedliche Bestandteile (z.B. Wasser, Fett, Proteine, etc.) eines Nahrungsmittels 130).

Durch die in diesem Dokument beschriebenen Maßnahmen wird Information über eine bevorzugte Verarbeitung eines Nahrungsmittels 130 ermittelt. Ferner kann ein Hausgerät 104 zur Verarbeitung eines Nahrungsmittels 130 automatisch an Eigenschaften des Nahrungsmittels 130 angepasst werden. So kann die Qualität eines gegarten Nahrungsmittels 130 erhöht werden.

Die vorliegende Erfindung ist nicht auf die gezeigten Ausführungsbeispiele beschränkt. Insbesondere ist zu beachten, dass die Beschreibung und die Figuren nur das Prinzip des vorgeschlagenen Systems und des vorgeschlagenen Verfahrens veranschaulichen sollen.

## Patentansprüche

1. System (100) zur Ermittlung von Information in Bezug auf ein Nahrungsmittel (130); wobei das System (100) umfasst:
- eine Zentraleinheit (101), insbesondere einen Server;
- ein elektronisches Anwendergerät (103), insbesondere ein Smartphone; und
- eine Vorrichtung (110) mit
- einer Messfläche (112) zur Ablage eines Nahrungsmittels (130);
- zumindest zwei an der Messfläche (112) angeordnete Messelektroden (121, 122, 123, 124), zwischen denen ein elektrisch isolierender Isoliersteg (125) verläuft;
- einer Kommunikationseinheit (113), die eingerichtet ist, über eine Kommunikationsverbindung (105) mit der Zentraleinheit (101) zu kommunizieren; und
- einer Steuereinheit (111), die eingerichtet ist,
- anhand der Messelektroden (121, 122, 123, 124) Sensordaten in Bezug auf das auf der Messfläche (112) abgelegte Nahrungsmittel (130) zu erfassen; und
- zu veranlassen, dass auf Basis der Sensordaten Nahrungsmittel-Information in Bezug auf eine Verarbeitung des Nahrungsmittels (130) ermittelt wird, wobei die Nahrungsmittel-Information anzeigt, wie ein Hausgerät bei der Verarbeitung des Nahrungsmittels einzustellen ist, und die Sensordaten zur Ermittlung der Nahrungsmittel-Information über die Kommunikationseinheit (113) an die Zentraleinheit (101) gesendet werden;
- wobei die Zentraleinheit (101) dafür eingerichtet ist, auf Basis der Sensordaten die Nahrungsmittel-Information in Bezug auf eine Verarbeitung des Nahrungsmittels (130) zu ermitteln und die Nahrungsmittel-Information an das elektronische Anwendergerät (103) zu senden.

2. System (100) gemäß Anspruch 1, wobei
- Kanten zumindest eines Teils der Messelektroden (121, 122, 123, 124) an den Isoliersteg (125) angrenzen;
- die Messelektroden (121, 122, 123, 124) jeweils eine Gesamt-Kantenlänge aufweisen; und
- ein an den Isoliersteg (125) angrenzender Anteil der Gesamt-Kantenlänge zumindest einer Messelektrode (121, 122, 123, 124) größer als 25% ist.

3. System (100) gemäß einem der vorhergehenden Ansprüche, wobei
- die Vorrichtung (110) einen Gewichtssensor (116) umfasst, der eingerichtet ist, Gewichtsdaten in Bezug auf das auf der Messfläche (112) abgelegte Nahrungsmittel (130) zu erfassen; und
- die Steuereinheit (111) eingerichtet ist, zu veranlassen, dass die Nahrungsmittel-Information auf Basis der Gewichtsdaten ermittelt wird und/oder das Erfassen der Sensordaten in Abhängigkeit von den Gewichtsdaten automatisch zu starten.

4. System (100) gemäß einem der vorhergehenden Ansprüche, wobei die Sensordaten umfassen,
- ein oder mehrere Messwerte in Bezug auf eine konduktive und/oder dielektrische Leitfähigkeit des Nahrungsmittels (130); und/oder
- Messwerte für eine Vielzahl von unterschiedlichen Frequenzen (211) einer an den Messelektroden (121, 122, 123, 124) angelegten Wechselspannung.

5. System (100) gemäß einem der vorhergehenden Ansprüche, wobei die Steuereinheit (111) eingerichtet ist, für eine Vielzahl von unterschiedlichen Frequenzen (211),
- eine Wechselspannung an den Messelektroden (121, 122, 123, 124) zu bewirken; und
- einen Messwert in Bezug auf eine Amplitude der Wechselspannung als Teil der Sensordaten zu erfassen.

6. System (100) gemäß einem der vorhergehenden Ansprüche, wobei
- die Vorrichtung (110) oder das Anwendergerät (103) eine Benutzerschnittstelle (114) umfasst; und
- die Steuereinheit (111) eingerichtet ist,
- anhand einer Nutzereingabe an der Benutzerschnittstelle (114) einen Nahrungsmitteltyp aus einer Mehrzahl von unterschiedlichen Nahrungsmitteltypen zu ermitteln; und
- zu veranlassen, dass die Nahrungsmittel-Information auf Basis des ermittelten Nahrungsmitteltyps ermittelt wird.

7. System (100) gemäß einem der vorhergehenden Ansprüche, wobei die Vorrichtung (110) einen elektrischen Energiespeicher (115) umfasst, der eingerichtet ist, elektrische Energie für einen autarken Betrieb der Vorrichtung (110) zu speichern.

8. System (100) gemäß einem der vorhergehenden Ansprüche, wobei
- die Vorrichtung (110) eine Vielzahl von Messelektroden (321, 121, 122, 123, 124) umfasst, die rasterförmig an der Messfläche (112) angeordnet ist; und
- die Steuereinheit (111) eingerichtet ist,
- einen Teilbereich der Messfläche (112) zu ermitteln, der von dem Nahrungsmittel (130) bedeckt ist;
- in Abhängigkeit von dem ermittelten Teilbereich der Messfläche (112) eine Teilmenge der Vielzahl von Messelektroden (321, 121, 122, 123, 124) auszuwählen; und
- die Sensordaten anhand der Teilmenge von Messelektroden (321, 121, 122, 123, 124) zu ermitteln.

9. System (100) gemäß einem der vorhergehenden Ansprüche, wobei die Zentraleinheit (101) eingerichtet ist, ein Hausgerät (104) zur Verarbeitung des Nahrungsmittels (130) automatisch in Abhängigkeit von der Nahrungsmittel-Information zu betreiben.

10. System (100) gemäß einem der vorhergehenden Ansprüche, wobei
- die Sensordaten Messwerte für eine Vielzahl von unterschiedlichen Frequenzen (211) einer an Messelektroden (121, 122, 123, 124) der Vorrichtung (110) angelegten Wechselspannung umfassen; und
- die Zentraleinheit (101) eingerichtet ist, anhand der Messwerte für unterschiedliche Frequenzen (211) Mengeninformation für unterschiedliche Bestandteile des Nahrungsmittels (130) zu ermitteln.

11. Verfahren (400) zur Ermittlung von Information in Bezug auf ein Nahrungsmittel (130) mittels eines Systems nach einem der vorhergehenden Ansprüche, wobei das Verfahren (400) umfasst,
- Erfassen (401), anhand der Messelektroden (121, 122, 123, 124), von Sensordaten in Bezug auf ein auf der Messfläche (112) abgelegtes Nahrungsmittel (130) mittels der Vorrichtung;
- Senden der Sensordaten zur Ermittlung der Nahrungsmittel-Information über die Kommunikationseinheit (113) der Vorrichtung und das Internet an die Zentraleinheit (101);
- Ermitteln auf Basis der Sensordaten von Nahrungsmittel-Information in Bezug auf eine Verarbeitung des Nahrungsmittels (130) mittels der Zentraleinheit (101), wobei die Nahrungsmittel-Information anzeigt, wie ein Hausgerät bei der Verarbeitung des Nahrungsmittels einzustellen ist; und
- Senden der Nahrungsmittel-Information von der Zentraleinheit (101) an das elektronische Anwendergerät (103).

## Claims

1. System (100) for determining information in relation to an item of food (130); wherein the system (100) comprises:
- a central unit (101), in particular a server;
- an electronic user device (103), in particular a smartphone; and
- an apparatus (110) having
- a measuring surface (112) for depositing an item of food (130);
- at least two measuring electrodes (121, 122, 123, 124) that are arranged on the measuring surface (112) and an electrically insulating bar (125) runs between said measuring electrodes;
- a communications unit (113) that is configured so as to communicate via a communication connection (105) with the central unit (101); and
- a control unit (111) that is configured
- so as to detect, with the aid of the measuring electrodes (121, 122, 123, 124), sensor data in relation to the item of food (130) that is deposited on the measuring surface (112); and
- so as to trigger, on the basis of the sensor data, a determination of food information in relation to a processing of the item of food (130), wherein the food information displays how a household appliance is to be set when the item of food is being processed, and the sensor data is transmitted via the communications unit (113) to the central unit (101) for the determination of the food information.
- wherein the central unit (101) is configured for the purpose of determining, on the basis of the sensor data, the food information in relation to processing the item of food (130) and so as to transmit the food information to the electronic user device (103).

2. System (100) according to claim 1, wherein
- edges of at least one part of the measuring electrodes (121, 122, 123, 124) adjoin the insulating bar (125);
- the measuring electrodes (121, 122, 123, 124) in each case have an overall edge length; and
- a portion of the overall edge length of at least one measuring electrode (121, 122, 123, 124) which adjoins the insulating bar (125) is greater than 25%.

3. System (100) according to one of the preceding claims, wherein
- the apparatus (110) comprises a weight sensor (116) that is configured so as to detect weight data in relation to the item of food (130) that is deposited on the measuring surface (112); and
- the control unit (111) is configured so as to trigger a determination of the food information on the basis of the weight data and/or so as to automatically start the detection of the sensor data in dependence upon the weight data.

4. System (100) according to one of the preceding claims, wherein the sensor data comprises
- one or multiple measurement values in relation to a conductive and/or dielectric conductivity of the item of food (130); and/or
- measurement values for a plurality of different frequencies (211) of an alternating current voltage that is applied to the measuring electrodes (121, 122, 123, 124).

5. System (100) according to one of the preceding claims, wherein the control unit (111) is configured for a plurality of different frequencies (211),
- so as to cause an alternating current voltage at the measuring electrodes (121, 122, 123, 124); and
- so as to detect a measurement value in relation to an amplitude of the alternating current voltage as part of the sensor data.

6. System (100) according to one of the preceding claims, wherein
- the apparatus (110) or the user device (103) comprises a user interface (114); and
- the control unit (111) is configured
- so as to determine, with the aid of a user input at the user interface (114), a food type from a plurality of different food types; and
- so as to trigger a determination of food information on the basis of the determined food type.

7. System (100) according to one of the preceding claims, wherein the apparatus (110) comprises an electrical energy storage device (115) that is configured so as to store electrical energy for an autarchic operation of the apparatus (110).

8. System (100) according to one of the preceding claims, wherein
- the apparatus (110) comprises a plurality of measuring electrodes (321, 121, 122, 123, 124) that is arranged in a grid-shaped manner on the measuring surface (112); and
- the control unit (111) is configured,
- so as to determine a part region of the measuring surface (112) that is covered by the food (130);
- so as to select, in dependence upon the determined part region of the measuring surface (112) a partial quantity of the plurality of measuring electrodes (321, 121, 122, 123, 124); and
- so as to determine the sensor data with the aid of the partial quantity of measuring electrodes (321, 121, 122, 123, 124).

9. System (100) according to one of the preceding claims, wherein the central unit (101) is configured so as to operate a household appliance (104) so as to process the item of food (130) automatically in dependence upon the food information.

10. System (100) according to one of the preceding claims, wherein
- the sensor data comprises measurement values for a plurality of different frequencies (211) of an alternating current voltage that is applied to measuring electrodes (121, 122, 123, 124) of the apparatus (110); and
- the central unit (101) is configured so as to determine, with the aid of the measurement values for different frequencies (211), quantity information for different elements of the food (130).

11. Method (400) for determining information in relation to an item of food (130) by means of a system as claimed in one of the preceding claims, wherein the method (400) comprises
- detecting (401) by means of the apparatus, with the aid of the measuring electrodes (121, 122, 123, 124), sensor data in relation to an item of food (130) that is deposited on the measuring surface (112);
- transmitting via the communication unit (113) of the apparatus and the internet to the central unit (101) the sensor data for the determination of the food information;
- determining, on the basis of the sensor data, food information in relation to a processing of the item of food (130) by means of the central unit (101), wherein the food information displays how a household appliance is to be set when the item of food is being processed; and
- transmitting the food information from the central unit (101) to the electronic user device (103).

## Revendications

1. Système (100) de détermination d'informations concernant une denrée alimentaire (130) ; dans lequel le système (100) comprend :
- une unité centrale (101), plus particulièrement un serveur ;
- un appareil utilisateur électronique (103), plus particulièrement un smartphone ; et
- un dispositif (110) avec
- une surface de mesure (112) pour déposer une denrée alimentaire (130) ;
- au moins deux électrodes de mesure (121, 122, 123, 124) disposées sur la surface de mesure (112), entre lesquelles passe une barrette isolante (125) isolante électriquement ;
- une unité de communication (113) qui est configurée pour communiquer avec l'unité centrale (101) par une liaison de communication (105) ; et
- une unité de commande (111) qui est configurée
- pour saisir à l'aide des électrodes de mesure (121, 122, 123, 124) des données de capteur concernant la denrée alimentaire (130) déposée sur la surface de mesure (112) ; et
- pour faire en sorte que sur la base des données de capteur soit déterminée l'information de denrée alimentaire concernant un traitement de la denrée alimentaire (130), l'information de denrée alimentaire indiquant comment régler un appareil ménager lors du traitement de la denrée alimentaire, et les données de capteur pour la détermination de l'information de denrée alimentaire sont envoyées à l'unité centrale (101) par l'unité de communication (113) ;
- dans lequel l'unité centrale (101) est configurée pour déterminer, sur la base des données de capteur, l'information de denrée alimentaire concernant un traitement de la denrée alimentaire (130) et envoyer l'information de denrée alimentaire à l'appareil utilisateur électronique (103).

2. Système (100) selon la revendication 1, dans lequel
- des bords d'au moins une partie des électrodes de mesure (121, 122, 123, 124) sont adjacents à la barrette isolante (125) ;
- les électrodes de mesure (121, 122, 123, 124) présentent à chaque fois une longueur globale de bords ; et
- une partie, adjacente à la barrette isolante (125), de la longueur globale de bords d'au moins une électrode de mesure (121, 122, 123, 124) est supérieure à 25 %.

3. Système (100) selon l'une des revendications précédentes, dans lequel
- le dispositif (110) comprend un capteur de poids (116) qui est configuré pour saisir des données de poids concernant la denrée alimentaire (130) déposée sur la surface de mesure (112) ; et
- l'unité de commande (111) est configurée pour faire en sorte que l'information de denrée alimentaire soit déterminée sur la base des données de poids et/ou pour débuter automatiquement la saisie des données de capteur en fonction des données de poids.

4. Système (100) selon l'une des revendications précédentes, dans lequel les données de capteur comprennent,
- une ou plusieurs valeurs de mesure concernant une conductivité conductive et/ou diélectrique de la denrée alimentaire (130) ; et/ou
- des valeurs de mesure pour une multitude de fréquences différentes (211) d'une tension alternative appliquée aux électrodes de mesure (121, 122, 123, 124).

5. Système (100) selon l'une des revendications précédentes, dans lequel l'unité de commande (111) est configurée, pour une multitude de fréquences différentes (211),
- pour provoquer une tension alternative sur les électrodes de mesure (121, 122, 123, 124) ; et
- pour saisir une valeur de mesure concernant une amplitude de la tension alternative en tant que partie des données de capteur.

6. Système (100) selon l'une des revendications précédentes, dans lequel le dispositif (110) ou l'appareil utilisateur (103) comprend une interface utilisateur (114) ; et
- l'unité de commande (111) est configurée,
- pour déterminer, à l'aide d'une entrée utilisateur à l'interface utilisateur (114), un type de denrée alimentaire parmi une pluralité de différents types de denrée alimentaire ; et
- pour faire en sorte que l'information de denrée alimentaire soit déterminée sur la base du type de denrée alimentaire déterminée.

7. Système (100) selon l'une des revendications précédentes, dans lequel le dispositif (110) comprend un réservoir d'énergie électrique (115) qui est configuré pour stocker de l'énergie électrique pour un fonctionnement autonome du dispositif (110).

8. Système (100) selon l'une des revendications précédentes, dans lequel
- le dispositif (110) comprend une pluralité d'électrodes de mesure (321, 121, 122, 123, 124) qui est disposée en forme de réseau sur la surface de mesure ; et
- l'unité de commande (111) est configurée
- pour déterminer une zone partielle de la surface de mesure (112), qui est recouverte de la denrée alimentaire (130) ;
- pour sélectionner une quantité partielle de la pluralité d'électrodes de mesure (321, 121, 122, 123, 124) en fonction de la zone partielle déterminée de la surface de mesure (112) ; et
- pour déterminer les données de capteur à l'aide de la quantité partielle d'électrodes de mesure (321, 121, 122, 123, 124).

9. Système (100) selon l'une des revendications précédentes, dans lequel l'unité centrale (101) est configurée pour faire fonctionner un appareil ménager (104) destiné au traitement de la denrée alimentaire (130) automatiquement en fonction de l'information de denrée alimentaire.

10. Système (100) selon l'une des revendications précédentes, dans lequel
- les données de capteur comprennent des valeurs de mesure pour une pluralité de fréquences différentes (211) d'une tension alternative appliquée aux électrodes de mesure (121, 122, 123, 124) du dispositif (110) ; et
- l'unité centrale (101) est configurée pour déterminer, à l'aide des valeurs de mesure pour des fréquences différentes (211), une information de quantités pour des parties constitutives différentes de la denrée alimentaire (130).

11. Procédé (400) de détermination d'informations concernant une denrée alimentaire (130) au moyen d'un système selon l'une des revendications précédentes, dans lequel le procédé (400) comprend,
- la saisie (401), à l'aide des électrodes de mesure (121, 122, 123, 124), de données de capteur concernant une denrée alimentaire (130) déposée sur la surface de mesure (112) au moyen du dispositif ;
- l'envoi des données de capteur pour déterminer l'information de denrée alimentaire à l'unité centrale (101) par l'unité de communication (113) du dispositif et par Internet ;
- la détermination sur la base des données de capteur de l'information de denrée alimentaire concernant un traitement de la denrée alimentaire (130) au moyen de l'unité centrale (101), l'information de denrée alimentaire indiquant la manière dont un appareil ménager doit être réglé lors du traitement de la denrée alimentaire ; et
- l'envoi de l'information de denrée alimentaire de l'unité centrale (101) à l'appareil utilisateur électronique (103).
